# FASCICULE DE BREVET EUROPEEN

(11) **EP 0 841 892 B1**
(45) Date de publication et mention de la délivrance du brevet: **28.11.2001**
(21) Numéro de dépôt: 96925803.7
(22) Date de dépôt: 17.07.1996
(51) Int. Cl.: A61K 7/06

(54) **COMPOSITION CAPILLAIRE A BASE DE MINOXIDIL A FAIBLE TENEUR EN SOLVANT GRAS**
MINOXIDIL ENTHALTENDES HAARPFLEGEMITTEL MIT EINEM NIEDRIGEN GEHALT AN FETTIGEM LÖSUNGSMITTEL
HAIR CARE COMPOSITION CONTAINING MINOXIDIL AND HAVING A LOW FATTY SOLVENT CONTENT

(30) Priorité: 18.07.1995 FR 9508659
(43) Date de publication de la demande: 20.05.1998
(73) Titulaire: Pierre Fabre Dermo-Cosmetique, 92100 Boulogne (FR)
(72) Inventeur: NAVARRO, Roger, F-09100 Pamiers (FR); DELAUNOIS, Marlène, F-31290 Villefranche-de-Lauragais (FR)
(74) Mandataire: Ahner, Francis
(86) Numéro de dépôt international: FR9601116
(87) Numéro de publication internationale: WO9703638

(56) Documents cités:
- EP-A- 0 624 599
- WO-A-95/25500
- FR-A- 2 602 421

## Description

L'invention concerne une composition capillaire à base de Minoxidil présentant une faible teneur en solvant gras pour stimuler la croissance des kéranocytes et favoriser la repousse des cheveux.

Il est bien connu aujourd'hui, notamment par le brevet US 4 139 619, que l'utilisation du Minoxidil par voie topique externe permet par traitement du cuir chevelu d'arrêter la chute des cheveux et de permettre leur repousse. De nombreux travaux cliniques ont démontré l'intérêt d'un tel produit et plusieurs spécialités ont été commercialisées à ce jour.

Le Minoxidil ou 6-(1-pipéridinyl)-2,4 pyrimidine diamine 3 oxyde, de formule : 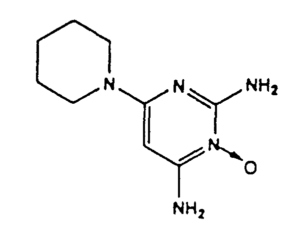 de par sa solubilité très faible en milieu aqueux, doit être associé à un mélange de solvants peu polaires afin de permettre une excellente solvatation et une bonne pénétration vers le bulbe pileux.

Dans le brevet américain précité, on a proposé d'utiliser des solvants du type glycol, comme le propylène glycol ou le polyéthylène glycol ou des solvants du type N-méthyl pyrolidone. Ces produits non volatils permettent de maintenir en solution le principe actif et assurer une bonne pénétration.

On a aussi décrit des formules utilisant des véhicules du type émulsion où le Minoxidil est soit dissous, soit dispersé sous forme de solution dans les produits plus lipophiles comme des esters de sorbitan ou des alcools gras éthoxylés.

Il existe aussi des onguents associant le Minoxidil à des corps gras comme la lanoline, les huiles de paraffine et la vaseline.

Cependant, ces solvants confèrent un caractère gras et non esthétique au cheveu et il est bien clair que ces formulations sont très difficiles à utiliser, car leur usage sur le cuir chevelu est très désagréable.

L'utilisateur lorsqu'il utilise ces lotions sur un long terme finit par cesser le traitement car si les résultats dans le domaine de la repousse sont remarquables, l'aspect huileux et non esthétique de la chevelure le font renoncer au traitement ou tout au moins espacer l'utilisation de la lotion et bien sûr en perdre les bénéfices.

Dans le but d'améliorer ces formulations, on a proposé des formulations associant des solvants de poids moléculaire variant de 200 à 600, comme le propylène glycol ou le polyéthylène glycol, la N-méthyl pyrolidone ou l'éther monoéthylique du diéthylène glycol, à l'alcool éthylique ou isopropylique. Ces solvants associés entre eux permettent de solubiliser le Minoxidil et d'assurer sa stabilité dans le temps. Ces formulations contiennent encore cependant des doses importantes de propylène glycol (30 %).

Il est bien connu que malgré la motivation de l'utilisateur, même les lotions associant alcool éthylique et propylène glycol qui sont considérées comme les produits les moins désagréables, sont abandonnées, car les cheveux sont rapidement gras, brillants et d'aspect peu esthétique.

Une solution évidente consisterait à remplacer le polyalcool par un autre alcool, mais la formulation d'une lotion alcool éthylique/eau conduit à l'insolubilité du Minoxidil à 2 % dans un mélange 50/50.

On mentionnera également la demande de brevet non publiée FR 94 03338 déposée le 22 Mars 1994 et relative à une composition capillaire contenant : 0,1 à 3 % en poids de Minoxidil,
0,1 à 3 % en poids de cyclodextrine,
0,5 à 10 % en poids d'un solvant du Minoxidil,
30 à 50 % en poids d'un alcool,
q.s.p. 100 % d'eau.

Les cyclodextrines explicitement mentionnées sont les α-cyclodextrines, les β-cyclodextrines, les β-cyclodextrines partiellement méthylées et les hydroxypropyl-β-cyclodextrines, seules ou en mélange.

La demande de brevet EP 0 624 599 décrit des nouveaux dérivés de mono-6-amino-6-désoxycyclodextrines, substitués en position 6 par un reste d'α-aminoacides qui présentent une meilleure solubilité et sont utilisés pour former des complexes d'inclusion de diverses substances actives.

La demande de brevet FR 2 602 421 décrit une composition sous forme de gel stimulant la croissance des cheveux et diminuant leur chute. Ces compositions peuvent comprendre du Minoxidil en solution dans un milieu aqueux contenant au moins un alcool inférieur en C₁C₄ et un agent gélifiant choisi parmi les hétérobiopolysaccharides et les dérivés de cellulose.

Par rapport aux lotions classiques qui utilisent des associations alcool/polyalcool, le but de cette invention est la réalisation d'une lotion capillaire agréable d'utilisation, tout en utilisant une quantité beaucoup plus faible de polyalcool, notamment le propylène glycol.

On a maintenant trouvé que les compositions capillaires à base de Minoxidil comprenant en tant que cyclodextrine, une γ-cyclodextrine permettrait de remplacer celles comprenant les cyclodextrines mentionnées dans la demande de brevet WO-A-95/25500 précitée.

La composition capillaire selon l'invention est caractérisée en ce qu'elle contient
0,1 à 7 % en poids de Minoxidil,
0,1 à 5 % en poids d'une γ-cyclodextrine,
0,5 à 15 % en poids d'un solvant du Minoxidil,
30 à 50 % en poids d'un mono-alcool,
q.s.p. 100 % d'eau.

Selon un autre aspect de la présente invention, la quantité de γ-cylodextrine présente dans la composition capillaire est telle qu'elle permet de diminuer substantiellement la quantité de solvant du Minoxidil qu'il serait normalement nécessaire d'ajouter pour obtenir une solubilité comparable du Minoxidil en absence de ladite cyclodextrine.

Par γ-cyclodextrine, on entend notamment des composés cycliques naturels consistant en 8 motifs (γ) (1→ 4) D-glucopyranosidiques, soit sous forme non substituée, soit sous forme substituée, par exemple par des groupes amino ou carboxy. Les γ-cyclodextrines peuvent être présentes seules ou en mélanges.

On préfèrera la γ-cyclodextrine non modifiée dont la solubilité dans l'eau est notamment de l'ordre de 23 %.

Les solvants du Minoxidil sont ceux qui sont généralement connus pour présenter une telle propriété, à savoir les dérivés glycoliques ou la N-méthyl pyrolidone ou les éthers monoéthyliques de diéthylène glycol.

Parmi les dérivés glycoliques, on citera à titre indicatif le propylène glycol, l'éthylène glycol, le dipropylène glycol et en général tous les polyéthylènes glycols de poids moléculaire variant de 200 à 600.

Le propylène glycol est néanmoins le glycol préféré pour la réalisation des compositions selon l'invention.

Parmi les mono-alcools, on peut citer les alcools en C₂-C₄ et notamment l'alcool éthylique ou l'alcool isopropylique.

Selon une première variante, la composition capillaire selon l'invention est caractérisée en ce qu'elle contient :
0,1 à 3 % en poids de Minoxidil,
0,1 à 3 % en poids d'une γ-cyclodextrine,
0,5 à 10 % en poids d'un solvant du Minoxidil,
30 à 50 % en poids d'un mono-alcool,
q.s.p. 100 % d'eau.

De préférence, la composition contient de 2 à 8 % de solvant du Minoxidil.

De préférence encore, la composition contient de 0,5 à 2,5 % en poids de Minoxidil.

Selon une seconde variante, la composition capillaire selon l'invention est caractérisée en ce qu'elle contient :
3 à 7 % en poids de Minoxidil,
2 à 5 % en poids d'une γ-cyclodextrine,
5 à 15 % en poids d'un solvant du Minoxidil,
30 à 50 % en poids d'un mono-alcool,
q.s.p. 100 % d'eau.

De préférence, la composition contient 8 à 12 % en poids d'un solvant du Minoxidil.

De préférence encore, la composition contient 4 à 6 % en poids de Minoxidil.

Lors d'essais effectués sur des patients en utilisant différentes lotions selon la présente invention, on a trouvé que les compositions capillaires selon l'invention présentaient des qualités particulièrement intéressantes après application, en ce qui concerne les trois aspects suivants :
- aspect non gras,
- temps de séchage de 5 minutes environ,
- facilité de coiffage.

Par ailleurs, 30 minutes après application, la chevelure présentait un aspect naturel, c'est-à-dire que les cheveux étaient "non gras", gonflants, non ternes, doux et faciles à coiffer.

Le lendemain des applications, ce même aspect était parfaitement conservé.

L'invention concerne également un procédé de traitement cosmétique contre l'alopécie consistant à appliquer une dose journalière efficace de Minoxidil sous la forme d'une composition telle que décrite précédemment.

La préparation des compositions selon l'invention s'effectue par mélange des différents ingrédients et plus précisément par dissolution du Minoxidil dans la phase alcoolique puis ajout de la γ-cyclodextrine, dudit solvant du Minoxidil et enfin de l'eau.

La lotion obtenue est limpide agréable à utiliser, non grasse et non collante.

Afin d'obtenir un séchage rapide de la composition et la meilleure tolérance possible, la composition doit de préférence avoir un degré alcoolique inférieur à 55 % en volume.

### Exemple 1

On réalise par dissolution de Minoxidil dans de l'alcool éthylique à 95 v/v puis ajout des autres ingrédients, une composition comprenant :
- Minoxidil 2 g
- γ-cyclodextrine (non modifiée) 1 g
- Alcool éthylique à 95 v/v 42,3 g
- Propylène glycol 5 g
- Eau purifiée qsp 100 ml.
- La γ-cyclodextrine présente une solubilité dans l'eau de 23 %.

### Exemple 2

On réalise selon le mode opératoire de l'exemple 1 la composition suivante :
- Minoxidil 1 g
- γ-cyclodextrine (non modifiée) 1 g
- Alcool éthylique à 95 v/v 40 g
- Propylène glycol 10 g
- Eau purifiée qsp 100 ml.
- La γ-cyclodextrine présente une solubilité dans l'eau de 23 %.

### Exemple 3

Selon le mode opératoire de l'exemple 1, on réalise la composition suivante :
- Minoxidil 5 g
- γ-cyclodextrine (non modifiée) 3 g
- Alcool éthylique à 95 v/v 42 g
- Propylène glycol 10 g
- Eau purifiée qsp 100 ml.
- La γ-cyclodextrine présente une solubilité dans l'eau de 23%.

### Exemple 4 (comparatif)

Selon le mode opératoire de l'exemple 1, on réalise la composition suivante :
. Minoxidil 2 g
. Alcool éthylique à 95 v/v 30 g
. Propylène glycol 30 g
. Eau purifiée qsp 100 ml.

### Exemple 5 - Essai sur le cuir chevelu

Les expériences décrites ci-après ont pour objet d'évaluer comparativement l'acceptabilité cosmétique et l'efficacité des compositions de lotion capillaire des exemples 1 à 4.

L'essai monocentrique a été effectué dans un centre spécialisé sur 12 sujets masculins en double aveugle, randomisé, réalisé en hémi-tête.
. L'essai a duré 8 jours.
. La quantité de composition appliquée par hémi-tête était de 0,5 ml.
   Les compositions des exemples 1, 2 et 3 ont un temps de séchage de cinq minutes environ et se sont révélées satisfaisantes en ce qui concerne l'aspect non gras de cheveux et la facilité de coiffage de ceux-ci. Ainsi, 30 minutes après l'application, la chevelure a un aspect naturel, les cheveux sont gonflants, "non gras", non ternes, doux, faciles à coiffer.
. Le lendemain des applications, ces cheveux restent "non gras", "non alourdis", gonflants, doux, brillants, faciles à coiffer, d'aspect naturel.

Les compositions des exemples 1 et 3 se sont révélées les plus intéressantes.

Les problèmes rencontrés avec la composition de l'exemple 4 sont les suivants :
. Temps de séchage supérieur à 30 minutes,
. Aspect "gras" des cheveux.

Les compositions des exemples 1, 2 et 3 selon l'invention se sont révélées, à quantité égale de Minoxidil, aussi efficaces que les mêmes compositions de l'art antérieur.

## Revendications

1. Composition capillaire pour stimuler la croissance des kératinocytes et favoriser la repousse des cheveux, **caractérisée en ce qu'**elle contient :
0,1 à 7 % en poids de Minoxidil,
0,1 à 5 % en poids d'une γ-cyclodextrine,
0,5 à 15 % en poids d'un solvant du Minoxidil,
30 à 50 % en poids d'un mono-alcool,
q.s.p. 100 % d'eau.

2. Composition selon la revendication 1, **caractérisée en ce qu'**elle contient :
0,1 à 3 % en poids de Minoxidil,
0,1 à 3 % en poids d'une γ-cyclodextrine,
0,5 à 10 % en poids d'un solvant du Minoxidil,
30 à 50 % en poids d'un mono-alcool,
q.s.p. 100 % d'eau.

3. Composition selon la revendication 2, **caractérisée en ce qu'**elle contient 2 à 8 % en poids d'un solvant du Minoxidil.

4. Composition selon l'une des revendications 2 ou 3, **caractérisée en ce qu'**elle contient entre 0,5 et 2,5 % en poids de Minoxidil.

5. Composition selon la revendication 1, **caractérisée en ce qu'**elle contient :
3 à 7 % en poids de Minoxidil,
2 à 5 % en poids d'une γ-cyclodextrine,
5 à 15 % en poids d'un solvant du Minoxidil,
30 à 50 % en poids d'un mono-alcool,
q.s.p. 100 % d'eau.

6. Composition selon la revendication 5, **caractérisée en ce qu'**elle contient 8 à 12 % en poids d'un solvant du Minoxidil.

7. Composition selon l'une des revendications 5 ou 6, **caractérisée en ce qu'**elle contient entre 4 à 6 % en poids de Minoxidil.

8. Composition selon l'une des revendications précédentes, **caractérisée en ce que** la γ-cyclodextrine est non modifiée.

9. Composition selon l'une des revendications précédentes, **caractérisée en ce que** le mono-alcool est choisi dans le groupe constitué par l'alcool éthylique et l'alcool isopropylique.

10. Composition selon l'une des revendications précédentes, **caractérisée en ce que** le solvant du Minoxidil est choisi parmi les polyéthylène glycols de poids moléculaire variant de 200 à 600.

11. Composition selon la revendication 11, **caractérisée en ce que** le solvant du Minoxidil est le propylène glycol.

## Claims

1. Composition for stimulating the growth of keratinocytes and for promoting hair regrowth, **characterized in that** it comprises:
0.1 to 7% by weight of minoxidil,
0.1 to 5% by weight of a γ-cyclodextrin,
0.5 to 15% by weight of a solvent of the minoxidil,
30 to 50% by weight of a monoalcohol,
q.s. for 100% of water.

2. Composition according to Claim 1, **characterized in that** it comprises:
0.1 to 3% by weight of minoxidil,
0.1 to 3% by weight of a γ-cyclodextrin,
0.5 to 10% by weight of a solvent of the minoxidil,
30 to 50% by weight of a monoalcohol,
q.s. for 100% of water.

3. Composition according to Claim 2, **characterized in that** it comprises 2 to 8% by weight of a solvent of the minoxidil.

4. Composition according to either of Claims 2 and 3, **characterized in that** it comprises between 0.5 and 2.5% by weight of minoxidil.

5. Composition according to Claim 1, **characterized in that** it comprises:
3 to 7% by weight of minoxidil,
2 to 5% by weight of a γ-cyclodextrin,
5 to 15% by weight of a solvent of the minoxidil,
30 to 50% by weight of a monoalcohol,
q.s. for 100% of water.

6. Composition according to Claim 5, **characterized in that** it comprises 8 to 12% by weight of a solvent of the minoxidil.

7. Composition according to either of Claims 5 and 6, **characterized in that** it comprises between 4 and 6% by weight of minoxidil.

8. Composition according to one of the preceding claims, **characterized in that** the γ-cyclodextrin is unmodified.

9. Composition according to one of the preceding claims, **characterized in that** the monoalcohol is chosen from the group consisting of ethyl alcohol and isopropyl alcohol.

10. Composition according to one of the preceding claims, **characterized in that** the solvent of the minoxidil is chosen from polyethylene glycols with a molecular weight varying from 200 to 600.

11. Composition according to Claim 10, **characterized in that** the solvent of the minoxidil is propylene glycol.

## Patentansprüche

1. Kapillarzusammensetzung zur Stimulierung des Wachstums von Keratinozyten und Förderung der Haarneubildung, **dadurch gekennzeichnet, dass** sie:
0,1 bis 7 Gew.-% Minoxidil,
0,1 bis 5 Gew.-% eines γ-Cyclodextrins,
0,5 bis 15 Gew.-% eines Lösungsmittels für Minoxidil,
30 bis 50 Gew.-% eines einwertigen Alkohols,
q.s.p. Wasser auf 100 %
enthält.

2. Zusammensetzung gemäß Anspruch 1, **dadurch gekennzeichnet, dass** sie
0,1 bis 3 Gew.-% Minoxidil,
0,1 bis 3 Gew.-% eines γ-Cyclodextrins,
0,5 bis 10 Gew.-% eines Lösungsmittels für Minoxidil,
30 bis 50 Gew.-% eines einwertigen Alkohols,
q.s.p. Wasser auf 100 %
enthält.

3. Zusammensetzung gemäß Anspruch 2, **dadurch gekennzeichnet, dass** sie 2 bis 8 Gew.-% eines Lösungsmittels für Minoxidil enthält.

4. Zusammensetzung gemäß Anspruch 2 oder 3, **dadurch gekennzeichnet, dass** sie zwischen 0,5 und 2,5 Gew.-% Minoxidil enthält.

5. Zusammensetzung gemäß Anspruch 1, **dadurch gekennzeichnet, dass** sie
3 bis 7 Gew.-% Minoxidil,
2 bis 5 Gew.-% eines γ-Cyclodextrins,
5 bis 15 Gew.-% eines Lösungsmittels für Minoxidil,
30 bis 50 Gew.-% eines einwertigen Alkohols,
g.s.p. Wasser auf 100 %
enthält.

6. Zusammensetzung gemäß Anspruch 5, **dadurch gekennzeichnet, dass** sie 8 bis 12 Gew.-% eines Lösungsmittels für Minoxidil enthält.

7. Zusammensetzung gemäß Anspruch 5 oder 6, **dadurch gekennzeichnet, dass** sie zwischen 4 und 6 Gew.-% Minoxidil enthält.

8. Zusammensetzung nach einem der vorhergehenden Ansprüche, **dadurch gekennzeichnet, dass** das γ-Cyclodextrin nicht modifiziert ist.

9. Zusammensetzung nach einem der vorhergehenden Ansprüche, **dadurch gekennzeichnet, dass** es sich bei dem einwertigen Alkohol um Ethylalkohol und/oder Isopropylalkohol handelt.

10. Zusammensetzung nach einem der vorhergehenden Ansprüche, **dadurch gekennzeichnet, dass** es sich bei dem Lösungsmittel für Minoxidil um Polyethylenglykole mit einem Molekulargewicht von 200 bis 600 handelt.

11. Zusammensetzung nach Anspruch 10, **dadurch gekennzeichnet, dass** es sich bei dem Lösungsmittel für Minoxidil um Propylenglykol handelt.
